(19) Europäisches Patentamt

European Patent Office

Office européen des brevets

(11) **EP 4 617 303 A1**

(12) **EUROPEAN PATENT APPLICATION**
published in accordance with Art. 153(4) EPC

(43) Date of publication:
**17.09.2025 Bulletin 2025/38**

(21) Application number: **24757251.4**

(22) Date of filing: **14.02.2024**

(51) International Patent Classification (IPC):
*C08J 3/24* (2006.01)    *C08J 3/12* (2006.01)
*C08F 20/10* (2006.01)    *C08L 33/04* (2006.01)
*C08L 25/08* (2006.01)    *C08L 25/14* (2006.01)
*C08K 5/56* (2006.01)    *C08K 5/00* (2006.01)
*C08K 5/09* (2006.01)    *C08K 5/17* (2006.01)

(52) Cooperative Patent Classification (CPC):
**C08F 20/10; C08J 3/12; C08J 3/24; C08K 5/00;
C08K 5/09; C08K 5/17; C08K 5/56; C08L 25/08;
C08L 25/14; C08L 33/04**

(86) International application number:
**PCT/KR2024/095142**

(87) International publication number:
**WO 2024/172530 (22.08.2024 Gazette 2024/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.02.2023 KR 20230019560
13.02.2024 KR 20240020382**

(71) Applicant: **LG CHEM, LTD.
Seoul 07336 (KR)**

(72) Inventor: **MIN, Ji Hong
Daejeon 34122 (KR)**

(74) Representative: **Plasseraud IP
104 Rue de Richelieu
CS92104
75080 Paris Cedex 02 (FR)**

(54) **SUPER ABSORBENT POLYMER AND PREPARATION METHOD THEREOF**

(57)    Provided are a superabsorbent polymer and a preparation method thereof, more particularly, a superabsorbent polymer, in which a specific deodorant is used to improve the deodorizing ability without deteriorating the inherent physical properties of the superabsorbent polymer, thereby effectively suppressing bad odors generated from urine, etc., when applied to products such as diapers, etc., and a preparation method thereof.

EP 4 617 303 A1

**Description**

[Technical Field]

Cross-reference to Related Application

The present application is based on, and claims priority from, Korean Patent Application Nos. 10-2023-0019560 and 10-2024-0020382, filed on February 14, 2023 and February 13, 2024, respectively, the disclosures of which are hereby incorporated by reference herein in their entirety.

The present invention relates to a superabsorbent polymer and a preparation method thereof. Particularly, the present invention relates to a superabsorbent polymer, in which a specific deodorant is used to improve the deodorizing ability without deteriorating the inherent physical properties of the superabsorbent polymer, thereby effectively suppressing bad odors generated from urine, etc., when applied to products such as diapers, etc., and a preparation method thereof.

[Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, and they are now being widely used not only for hygiene products such as disposable diapers for children, etc., but also for water retaining soil products for gardening, water stop materials for the civil engineering and construction, sheets for raising seedling, fresh-keeping agents for food distribution fields, materials for poultices, or the like.

In most cases, these superabsorbent polymers have been widely used in the field of sanitary materials such as diapers or sanitary napkins, etc. Inside the sanitary materials, the superabsorbent polymer is generally distributed throughout pulp. However, recent efforts have been continuously made to provide sanitary materials such as diapers having a thinner thickness, etc., and as part of that, diapers having a reduced content of pulp, and furthermore, diapers having no pulp, so-called pulpless diapers are actively under development.

As described above, such a sanitary material having a reduced content of pulp or having no pulp includes the superabsorbent polymer at a relatively high ratio, and the superabsorbent polymer particles are inevitably included as multiple layers in the sanitary materials. In order to allow the whole superabsorbent polymer particles included as multiple layers to more efficiently absorb a large amount of liquid such as urine, etc., it is necessary that the superabsorbent polymer basically exhibits high absorption performance and high absorption rate. Further, the superabsorbent polymer should not release absorbed liquid even under an external pressure, and furthermore, requires liquid permeability to well retain its original shape even in a swollen state by absorbing liquid. Accordingly, in order to improve the basic water absorption and water retention capacity of the superabsorbent polymer, much research is being conducted, such as surface crosslinking, etc.

Meanwhile, the superabsorbent polymer may be used in sanitary materials such as diapers, sanitary napkins, etc., and in this case, there may be a problem of reduced usability due to bad odor of the absorbed liquid, such as human and pet excrement.

Accordingly, there is an increasing demand for not only absorption capacity and absorption rate, which are the basic physical properties of the superabsorbent polymer, but also bad odor suppression. Therefore, it is necessary to prepare a superabsorbent polymer capable of effectively suppressing bad odor.

[Disclosure]

[Technical Problem]

Accordingly, there are provided a superabsorbent polymer, in which a specific deodorant is used to improve the deodorizing ability without deteriorating the inherent physical properties of the superabsorbent polymer, thereby effectively suppressing bad odors generated from urine, etc., when applied to products such as diapers, etc., and a preparation method thereof.

[Technical Solution]

To achieve the above objects, according to one embodiment of the present invention, there is provided a method of preparing a superabsorbent polymer, the method including the steps of:

forming a hydrogel polymer by performing a polymerization on a monomer composition including an acrylic acid-based monomer having acidic groups of which at least part is neutralized, an internal crosslinking agent, and a polymerization initiator (step 1);

forming a base resin by drying, pulverizing, and classifying the hydrogel polymer (step 2);

mixing the base resin and a deodorant (step 3); and

performing a surface crosslinking reaction on the mixture of the base resin and the deodorant in the presence of a surface crosslinking solution including a surface crosslinking agent (step 4),

wherein the deodorant includes a metal organic framework (MOF) or an ion exchange resin.

According to another embodiment of the present invention, there is provided a superabsorbent polymer including:

a base resin complex including a base resin, in which an acrylic acid-based monomer having acidic groups of which at least part is neutralized, and an internal crosslinking agent are polymerized, and at least part of a deodorant which is supported on the polymer chains of the base resin; and

a surface crosslinked layer which is formed on the surface of the base resin,

wherein the deodorant includes a metal organic framework (MOF) or an ion exchange resin.

[Effect of the Invention]

As described above, the present invention uses a metal organic framework (MOF) or an ion exchange resin as a deodorant to improve the deodorizing ability without deteriorating the inherent physical properties of a superabsorbent polymer, thereby effectively suppressing bad odors generated from urine, etc., when applied to products such as diapers, etc.

[Best Mode for Carrying Out the Invention]

The terms used in this description are just for explaining exemplary embodiments and it is not intended to restrict the present invention.

The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components, or combinations thereof beforehand.

The terms "first, second, third", and the like are used to describe a variety of components, and these terms are merely employed to differentiate a certain component from other components.

As used herein, the term "polymer" refers to those in a polymerized state of an acrylic acid-based monomer, and may include all water content ranges or particle size ranges. Among the above polymers, a polymer having a water content (moisture content) of about 40% by weight or more in a state after polymerizing before drying may be referred to as a hydrogel polymer, and particles obtained by pulverizing and drying such a hydrogel polymer may be referred to as a crosslinked polymer.

Further, the term "base resin" or "base resin powder" refers to particles or powders prepared by drying and pulverizing a polymer of acrylic acid-based monomers, and it means a polymer on which the surface-modification or surface-crosslinking step as explained later is not performed.

Further, the term "superabsorbent polymer" or "superabsorbent polymer powder" refers to, depending on the context, a crosslinked polymer obtained by polymerizing a water-soluble ethylenically unsaturated monomer (acrylic acid-based monomer) including acidic groups of which at least part is neutralized, or a base resin in the form of powder consisting of superabsorbent polymer particles obtained by pulverizing the crosslinked polymer, or is used to encompass those made suitable for commercialization by performing an additional process on the crosslinked polymer or the base resin, for example, surface crosslinking, re-assembling of fine particles, drying, pulverizing, classifying, etc.

The present invention may be variously modified and have various forms, and specific exemplary embodiments are exemplified and explained in detail in the following description. However, it is not intended to limit the present invention to the specific exemplary embodiments, and it must be understood that the present invention includes every modifications, equivalents, or replacements included in the spirit and technical scope of the present invention.

Hereinafter, a method of preparing a superabsorbent polymer and a superabsorbent polymer will be described in more detail according to specific embodiments of the present invention.

A method of preparing a superabsorbent polymer according to one embodiment of the present invention includes the steps of:

forming a hydrogel polymer by performing a polymerization on a monomer composition including an acrylic acid-based monomer having acidic groups of which at least part is neutralized, an internal crosslinking agent, and a polymerization initiator (step 1);

forming a base resin by drying, pulverizing, and classifying the hydrogel polymer (step 2);

mixing the base resin and a deodorant (step 3); and

performing a surface crosslinking reaction on the mixture of the base resin and the deodorant in the presence of a surface crosslinking solution including a surface crosslinking agent (step 4),

wherein the deodorant includes a metal organic framework (MOF) or an ion exchange resin.

The superabsorbent polymer is used in a variety of sanitary materials such as diapers, sanitary napkins, etc., but when actually used, the usability may be reduced due to bad odor of excrement from human and pet. Further, as the wearing time elapses, there is a problem that the growth of bacteria is accelerated by the liquid absorbed into the product, resulting in additional bad odor generation.

Deodorizing substances traditionally included in products for bad odor reduction have problems that excessive amounts thereof must be used to achieve the desired level of deodorizing ability, and accordingly, absorption properties are significantly reduced and the cost of the product increases.

Accordingly, the present inventors found that when a metal organic framework (MOF) or an ion exchange resin is used as a deodorant, and instead of simply mixing the same with a final superabsorbent polymer, the deodorant is mixed with a base resin before surface crosslinking, and then surface crosslinking is performed, bad odors generated by various causes may be effectively controlled while maintaining the white color without deteriorating the basic physical properties of the superabsorbent polymer, such as water retention capacity, absorption properties such as absorbency under pressure, and absorption rate, thereby completing the present invention.

Hereinafter, each step of the method of preparing a superabsorbent polymer according to one embodiment of the present invention will be described.

**(Step 1)**

The step 1 is a step of preparing a hydrogel polymer, specifically, a step of forming the hydrogel polymer by performing a polymerization on a monomer composition including an acrylic acid-based monomer having acidic groups of which at least part is neutralized, an internal crosslinking agent, and a polymerization initiator.

The acrylic acid-based monomer may be any monomer commonly used in the preparation of superabsorbent polymers. Specifically, the acrylic acid-based monomer may be a compound represented by the following Chemical Formula 1:

[Chemical Formula 1]          $R^1-COOM^1$

in Chemical Formula 1,

$R^1$ is a C2-C5 alkyl group containing an unsaturated bond, and

$M^1$ is a hydrogen atom, a monovalent or divalent metal, an ammonium group, or an organic amine salt.

Preferably, the acrylic acid-based monomer includes one or more selected from the group consisting of acrylic acid, methacrylic acid, and a monovalent metal salt thereof, a divalent metal salt thereof, an ammonium salt thereof, and an organic amine salt thereof.

The acrylic acid-based monomer has acidic groups of which at least part may be neutralized. Preferably, those partially neutralized with an alkali material such as sodium hydroxide, potassium hydroxide, ammonium hydroxide, etc. may be used as the monomer.

In this regard, a degree of neutralization of the monomer may be 40 mol% to 95 mol%, or 40 mol% to 80 mol%, or 45 mol% to 75 mol%. The range of the degree of neutralization may vary depending on the final physical properties. However, when the degree of neutralization is excessively high, the neutralized monomers are precipitated, and thus polymerization may not readily occur. On the contrary, when the degree of neutralization is excessively low, absorbency of the polymer greatly decreases, and furthermore, the polymer may exhibit hard-to-handle properties, like elastic rubber.

The "internal crosslinking agent" is a term used to distinguish it from a "surface crosslinking agent" for crosslinking the surface of the base resin, and the internal crosslinking agent functions to polymerize the acrylic acid-based monomers by crosslinking the unsaturated bonds thereof. The crosslinking in the above step occurs regardless of the surface or inside of the polymer. However, through the surface crosslinking process of the base resin described below, the particle surface of the superabsorbent polymer finally prepared has a structure crosslinked by the surface crosslinking agent, and the inside thereof has a structure crosslinked by the internal crosslinking agent.

As the internal crosslinking agent, any compound may be used as long as it enables the introduction of crosslinking bonds during polymerization of the acrylic acid-based monomer. For non-limiting examples of the internal crosslinking agent, multifunctional crosslinking agents such as N,N'-methylenebisacrylamide, trimethylolpropane tri(meth)acrylate, ethylene glycol di(meth)acrylate, polyethylene glycol di(meth)acrylate, propylene glycol di(meth)acrylate, polypropylene glycol di(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth) acrylate, tetraethylene glycol di(meth)acrylate, dipentaerythritol pentaacrylate, glycerin tri(meth)acrylate, pentaerythritol tetraacrylate, triallylamine, allyl (meth)acrylate, ethylene glycol diglycidyl ether, propylene glycol, glycerin, or ethylene carbonate may be used alone or in combination of two or more thereof.

Such an internal crosslinking agent may be added at a concentration of 0.001% by weight to 1% by weight, or 0.01% by weight to 0.8% by weight, or 0.1% by weight to 0.7% by weight with respect to the monomer composition. In other words, when the concentration of the internal crosslinking agent is excessively low, the absorption rate of the resin may decrease, and the gel strength may become weak, which is not preferred. On the contrary, when the concentration of the internal crosslinking agent is excessively high, the absorption capacity of the resin may decrease, which is not preferred as an absorber.

In addition, the monomer composition may further include additives such as thickeners, plasticizers, storage stabilizers, and antioxidants, etc., as needed.

Further, the monomer composition may include a polymerization initiator which is generally used in the preparation of superabsorbent polymers.

As the polymerization initiator, a thermal polymerization initiator or a photopolymerization initiator may be used depending on a polymerization method. However, even though the photopolymerization is performed, a certain amount of heat may be generated by UV irradiation or the like, and also generated with the polymerization reaction which is an exothermic reaction. Therefore, the thermal polymerization initiator may be further included.

Here, as the photopolymerization initiator, for example, one or more compounds selected from the group consisting of benzoin ether, dialkyl acetophenone, hydroxyl alkylketone, phenyl glyoxylate, benzyl dimethyl ketal, acyl phosphine, and $\alpha$-aminoketone may be used. Among them, specific examples of acyl phosphine may include commercially available lucirin TPO, i.e., 2,4,6-trimethyl-benzoyl-trimethyl phosphine oxide. More various photopolymerization initiators are disclosed in "UV Coatings: Basics, Recent Developments and New Application(Elsevier, 2007)" written by Reinhold Schwalm, p115, which may be served as a reference.

As the thermal polymerization initiator, one or more compounds selected from the group consisting of persulfate-based initiators, azo-based initiators, hydrogen peroxide, and ascorbic acid may be used. Specifically, the persulfate-based initiators may be exemplified by sodium persulfate ($Na_2S_2O_8$), potassium persulfate ($K_2S_2O_8$), ammonium persulfate (($NH_4)_2S_2O_8$) or the like. Further, the azo-based initiators may be exemplified by 2,2-azobis(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane] dihydrochloride, 4,4-azobis-(4-cyanovaleric acid) or the like. More various thermal polymerization initiators are disclosed in "Principle of Polymerization(Wiley, 1981)" written by Odian, p203, which may be served as a reference.

Such a polymerization initiator may be added at a concentration of 0.001% by weight to 1% by weight, or 0.005% by weight to 0.1% by weight with respect to the monomer composition. In other words, when the concentration of the polymerization initiator is too low, the polymerization rate may become slow, and monomers remaining in the final product may be extracted in a large amount, which is not preferred. On the contrary, when the concentration of the polymerization initiator is too high, polymer chains constituting the network become short, and thus the content of water-soluble components is increased and physical properties of the resin may deteriorate, such as a reduction in absorbency under pressure, which is not preferred.

Further, such a monomer composition may be prepared in a solution form, in which the raw materials, such as the above-described acrylic acid-based monomer, polymerization initiator, internal crosslinking agent, blowing agent, etc., are dissolved in a solvent.

In this regard, as the applicable solvent, any solvent may be used without limitations on the composition, as long as it is able to dissolve the above-described raw materials. For example, as the solvent, water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, N,N-dimethylacetamide, or a mixture thereof may be used.

The formation of the hydrogel polymer through the polymerization of the monomer composition may be performed by a common polymerization method, and the process is not particularly limited.

For non-limiting example, the polymerization method may be classified into thermal polymerization and photo-polymerization according to a polymerization energy source, and when the thermal polymerization is carried out, it may be carried out in a reactor like a kneader equipped with agitating spindles, and when the photo-polymerization is carried out, it may be carried out in a reactor equipped with a movable conveyor belt.

For example, the monomer composition is injected to the reactor like the kneader equipped with the agitating spindles, and thermal polymerization is carried out by providing hot air thereto or by heating the reactor, thereby obtaining the hydrogel polymer. In this regard, the hydrogel polymer discharged from an outlet of the reactor may be obtained as particles having a size of several centimeters or millimeters, according to the type of agitating spindles equipped in the reactor. Specifically, the hydrogel polymer may be obtained in various forms according to a concentration of the monomer composition fed thereto, a feeding speed or the like, and the hydrogel polymer having a (weight average) particle size of 2 mm to 50 mm may be generally obtained.

For another example, when the monomer composition is subjected to photopolymerization in the reactor equipped with the movable conveyor belt, the hydrogel polymer may be obtained in a sheet-type. In this regard, the thickness of the sheet may vary according to the concentration of the monomer composition fed thereto and the feeding speed. The sheet is preferably controlled at a thickness of 0.5 cm to 10 cm in order to assure the production speed while allowing the entire sheet to be uniformly polymerized.

The hydrogel polymer thus obtained by such a method may exhibit a water content of 40% by weight to 80% by weight. Here, the water content means a weight occupied by water with respect to the total weight of the hydrogel polymer, which may be a value obtained by subtracting the weight of the dried polymer from the weight of the hydrogel polymer. Specifically, the water content may be defined as a value calculated by measuring the weight loss due to evaporation of water in the polymer during the process of drying by raising the temperature of the polymer through infrared heating. At this time, the drying conditions may be set as follows: the temperature is increased from room temperature to 180°C and then the temperature is maintained at 180°C, and the total drying time is set to 20 minutes, including 5 minutes for the temperature rising step.

**(Step 2)**

The step 2 of the present invention is a step of forming a base resin powder by drying, pulverizing, and classifying the hydrogel polymer prepared in the step 1.

The step of coarsely pulverizing the hydrogel polymer may be further included, before drying the same. Hereinbelow, in order to distinguish it from pulverizing after drying, the term 'coarsely pulverizing' is used herein for convenience in relation to pulverizing before drying.

A pulverizer to be used for the pulverizing may include, but there is no limitation in the configuration, specifically, any one selected from the group consisting of a vertical pulverizer, a turbo cutter, a turbo grinder, a rotary cutter mill, a cutter mill, a disc mill, a shred crusher, a crusher, a chopper, and a disc cutter, but is not limited to the above-described examples.

At this time, the step of coarsely pulverizing may be performed such that the hydrogel polymer has a particle size of about 2 mm to about 10 mm. Pulverizing the hydrogel polymer into a particle size of less than 2 mm is technically not easy due to a high water content of the hydrogel polymer, and a phenomenon of agglomeration may occur between the pulverized particles. In contrast, when the hydrogel polymer is pulverized into a particle size of larger than 10 mm, the effect of increasing the efficiency in the subsequent drying step may be poor.

The drying may be performed at a temperature of 120°C to 250°C, 140°C to 200°C, or 150°C to 190°C. At this time, the drying temperature may be defined as the temperature of a heating medium supplied for drying or the internal temperature of a drying reactor containing the heating medium and the polymer in the drying process. When the drying temperature is low and the drying time is long, the process efficiency decreases. To prevent this, the drying temperature is preferably 120°C or higher. Further, when the drying temperature is higher than necessary, the surface of the hydrogel polymer may be excessively dried, which may increase the generation of fine powder in the subsequent pulverizing step, and the physical properties of the final resin may deteriorate. To prevent this, the drying temperature is preferably 250°C or lower.

At this time, the drying time in the drying step is not particularly limited, but may be adjusted to 20 minutes to 90 minutes under the drying temperature, considering the process efficiency and physical properties of the resin.

The drying may be performed using a common medium, for example, may be performed through methods such as supplying hot air, infrared irradiation, microwave irradiation, or ultraviolet irradiation for the pulverized hydrogel polymer, etc.

Further, the drying is preferably performed such that the dried polymer may have a water content of 0.1% by weight to 10% by weight. In other words, when the water content of the dried polymer is less than 0.1% by weight, excessive drying may increase the production costs and may cause degradation of the crosslinked polymer, which is not preferred. Further, when the water content of the dried polymer is more than 10% by weight, defects may occur in subsequent processes, which is not preferred.

Subsequently, the dried hydrogel polymer may be pulverized. This is a step to optimize the surface area of the base resin powder and the superabsorbent polymer. The pulverization may be performed so that the particle size of the pulverized polymer is 150 $\mu$m to 850 $\mu$m.

In this regard, a pulverizer to be applicable may include common pulverizers such as a pin mill, a hammer mill, a screw mill, a roll mill, a disc mill, or a jog mill, etc.

Further, in order to manage the physical properties of the superabsorbent polymer to be finally commercialized, a step of selectively classifying particles having a particle size of 150 $\mu$m to 850 $\mu$m from the polymer particles, which are obtained through the pulverizing step, may be further performed.

Through the above classification step, a base resin powder may be obtained. This base resin powder may have a particle size of 150 $\mu$m to 850 $\mu$m, and may include 2% by weight or less or 1% by weight or less of fine powder having a particle size of less than 150 $\mu$m.

**(Step 3)**

The step 3 of the present invention is a step of mixing the base resin with a metal organic framework (MOF) or an ion exchange resin as a deodorant.

The metal-organic framework (MOF), also called metal-organic structure, is an organic-inorganic hybrid material in which metal ions or ion clusters are coordinated with organic ligands to form primary, secondary or tertiary structures, and various MOFs may be prepared according to the selection of metal ions and organic ligands.

The MOFs are characterized by being porous, in which empty spaces exist within the structure, and the pore size, porosity, three-dimensional structure, surface area, etc. may be variously designed depending on the type of metal ions and organic ligands that constitute the MOF and a binding method.

Due to this porosity, MOF has adsorption properties for various organic compounds. In particular, as compared to the widely known zeolite or activated carbon, MOF has excellent adsorption properties for various substances, and thus is receiving attention as a next-generation functional adsorbent. In addition, the MOF does not easily deform at high temperatures and has a strong skeleton, and thus it has excellent chemical and thermal stability. Further, some of the MOFs are known to have antibacterial properties against microorganisms.

Meanwhile, the inventors of the present invention confirmed that when this MOF is included in a superabsorbent polymer, it is harmless to the human body without impairing the inherent properties of the superabsorbent polymer, and has excellent deodorizing ability to suppress various odorous substances.

According to one exemplary embodiment of the present invention, the MOF may include one or more metal ions selected from the group consisting of Zn, Ti, Co, Al, and Zr, and one or more organic ligands selected from the group consisting of imidazole, alkylimidazole, alkoxyimidazole, terephthalic acid, and aminoterephthalic acid.

According to one exemplary embodiment of the present invention, the MOF may include, for example, one or more selected from the group consisting of Zn-MOF (ZIF-8, ($Zn^{2+}$+2-methylimidazole)), Zn-MOF (ZIF-67, ($Co^{2+}$+2-methylimidazole)), Ti-MOF ($Tn^{2+}$+2-methylimidazole), $NH_2$-UiO-66($Zr^{4+}$+2-aminoterephthalic acid), and $NH_2$-MI-L-101($Al^{3+}$+2-aminoterephthalic acid), but the present invention is not limited thereto.

It was also confirmed that when the ion exchange resin is included in a superabsorbent polymer, it is harmless to the human body without impairing the inherent properties of the superabsorbent polymer, and has excellent deodorizing ability to suppress various odorous substances.

According to one exemplary embodiment of the present invention, as for the ion exchange resin, a hydrophobic ion exchange resin is generally preferred, which is advantageous in removing hydrophobic odorous components. For example, a polystyrene-divinylbenzene-based, polymethacrylate-divinylbenzene-based, or polyacrylate-divinylbenzene-based resin may be used. More specifically, for example, the ion exchange resin may include one or more selected from the group consisting of commercially available Amberlite XAD, Samyang Trilite, Samyang DIAION, etc., but the present invention is not limited thereto.

The deodorants may be respectively included in an amount of 1 part by weight to 10 parts by weight with respect to 100 parts by weight of the base resin. Specifically, the MOF or ion exchange resin may be included in an amount of 1 part by weight or more, 2 parts by weight or more, 3 parts by weight or more, 4 parts by weight or more, or 5 parts by weight or more, and 10 parts by weight or less with respect to 100 parts by weight of the base resin. When the deodorant is used in the above range of content, the deodorizing ability of the superabsorbent polymer may be further improved without deteriorating the absorption properties.

Meanwhile, when the MOF or ion exchange resin is simply mixed with the final superabsorbent polymer by a post-process after surface crosslinking, there is a problem in that the deodorizing effect is not maintained for a long time because it is easily desorbed from the superabsorbent polymer. In contrast, according to one embodiment of the present invention, the base resin and the deodorant are mixed before surface crosslinking, and then surface crosslinking is performed, and accordingly, their bonded state is maintained without being easily desorbed until the product is finished into an article such as diapers, etc., and when wet by urine which causes bad odor, the resin expands to desorb the deodorant which is then widely distributed, thereby exerting deodorizing ability. Accordingly, the deodorizing effect of effectively controlling bad odors due to various causes will be maintained for a long time while maintaining the white color without deteriorating the basic physical properties of the superabsorbent polymer, such as water retention capacity, the absorption properties, such as absorbency under pressure, and absorption rate.

On the other hand, it was observed that when the deodorant is mixed with the water-containing gel before preparing the base resin, there is a problem of loss of deodorizing ability, such as the pores in the deodorant being clogged with moisture due to the large amount of moisture present on the surface of the water-containing gel. Therefore, it is preferable that the MOF or ion exchange resin as the deodorant is mixed with the base resin which is not in a water-containing gel state but has been dried to have a water content of 10% or less.

**(Step 4)**

The step 4 of the present invention is a step of performing a surface crosslinking reaction on the mixture of the base resin and the deodorant, which is obtained in the step 3, in the presence of a surface crosslinking solution including a surface crosslinking agent.

A surface crosslinked layer is formed on the surface of the base resin particles by the step 4.

The formation of the surface crosslinked layer may be performed by a common method of increasing the crosslinking density of the surface of polymer particle, for example, by a method of performing a crosslinking reaction by mixing the pulverized polymer with the surface crosslinking solution including the surface crosslinking agent and then heat-treating the same.

The surface crosslinking step may be performed at a temperature of about 80°C to about 250°C. More specifically, the surface crosslinking process may be performed at a temperature of about 100°C to about 220°C, or about 110°C to about 200°C, or about 120°C to about 190°C for about 10 minutes to about 2 hours, or about 20 minutes to about 60 minutes. The crosslinking reaction temperature is lower than 160°C or the reaction time is too short, the surface crosslinking reaction does not occur properly and thus permeability may be lowered. When the temperature is higher than 200°C or the reaction time is too long, there may be a problem that the water retention capacity may be reduced.

A heating means for the surface crosslinking reaction is not particularly limited. Heating may be performed by providing a heating medium or by directly providing a heat source. In this regard, the kind of the applicable heating medium may be steam, hot air, a hot fluid such as hot oil or the like, but the present invention is not limited thereto. The temperature of the heating medium to be provided may be properly selected in consideration of the means of the heating medium, the heating rate, and the heating target temperature. Meanwhile, as the heat source to be directly provided, an electric heating or gas heating method may be used, but the present invention is not limited to the above-described examples.

The surface crosslinking solution includes a surface crosslinking agent, and the surface crosslinking agent is a surface crosslinking agent generally used for surface crosslinking of superabsorbent polymers. There is no particular limitation as long as it is a compound capable of reacting with a functional group of the polymer.

Preferably, in order to improve the properties of superabsorbent polymer to be prepared, as the surface crosslinking agent, one or more selected from the group consisting of polyhydric alcohol; epoxy compounds; polyamine compounds; haloexpoy compounds; condensation products of haloexpoy compounds; oxazoline compounds; mono-, di- or polyoxazolidinone compounds; cyclic urea compounds; multivalent metal salts; and alkylene carbonate compounds may be used.

Specifically, as the examples of the polyhydric alcohol compounds, one or more selected from the group consisting of mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexandimethanol may be used.

Further, as the epoxy compounds, ethylene glycol diglycidyl ether and glycidol, etc. may be used, and as the polyamine compounds, one or more selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compounds, epichlorohydrin, epibromohydrin, and $\alpha$-methylepichlorohydrin may be used. Meanwhile, as the mono-, di- or polyoxazolidinone compounds, for example, 2-oxazolidinone may be used.

Further, as the alkylene carbonate compounds, ethylene carbonate, etc. may be used. These compounds may be used alone or in combinations. Meanwhile, in order to increase the efficiency of the surface crosslinking process, it is preferable to include one or more C2 to C10 polyhydric alcohol compounds among the surface crosslinking agents.

The content of the surface crosslinking agent to be added may be appropriately selected according to the kind of surface crosslinking agents or reaction conditions, but commonly, it may be used in an amount of about 0.001 part by weight to about 5 parts by weight, preferably, about 0.01 part by weight to about 3 parts by weight, and more preferably, about 0.05 parts by weight to about 2 parts by weight with respect to 100 parts by weight of the polymer.

When the content of the surface crosslinking agent is too small, a surface crosslinking reaction may hardly occur, and when the content exceeds 5 parts by weight with respect to 100 parts by weight of the polymer, absorption capacity and physical properties may deteriorate, due to progression of excessive surface crosslinking reaction.

Meanwhile, the surface crosslinking solution may further include inorganic materials. As such inorganic materials, one or more inorganic materials selected from the group consisting of silica, clay, alumina, silica-alumina composite, titania, zinc oxide, and aluminum sulfate may be used. The inorganic material may be used in the form of powder or liquid, and particularly, in the form of alumina powder, silica-alumina powder, titania powder, or a nanosilica solution. Further, the inorganic material may be used in an amount of about 0.001 part by weight to about 1 part by weight with respect to 100 parts by weight of the base resin.

In the surface crosslinking solution, water may be used as a solvent. In this regard, the content of water may be properly controlled in order to induce uniform dispersion of the surface crosslinking agent and the deodorant, to prevent the aggregation phenomenon of the base resin powder, and at the same time, to optimize the surface penetration depth of the crosslinking agent.

Upon performing the surface crosslinking reaction when the base resin and the deodorant are physically mixed, the base resin expands due to the surface crosslinking solution, and some of the deodorant particles may physically attach to the base resin. In other words, at least part of the deodorant particles are stuck to be supported in the polymer chains of the base resin. As a result, the bonded state is maintained without being easily detached until subsequent processes such as the surface crosslinking step, etc. or the completion of products such as diapers, etc., and when wet by urine which causes bad odor, the resin expands to desorb the deodorant which is then widely distributed, thereby exerting deodorizing ability.

Meanwhile, according to one embodiment of the present invention, a chelating agent or an organic acid may be further mixed at or after the surface crosslinking step, in order to improve the deodorizing ability.

The chelating agent may include one or more selected from the group consisting of ethylenediaminetetraacetic acid (EDTA), L-glutamic acid diacetic acid (GLDA), methyl glycine diacetic acid (MGDA), hydroxyethyl ethylenediaminetriacetic acid (HEDTA), ethanol diglycinic acid (EDG), diethylenetriaminepentaacetic acid (DTPA), and salts thereof. More specifically, the chelating agent may be ethylenediaminetetraacetic acid (EDTA), or L-glutamic acid diacetic acid (GLDA).

The organic acid may be one or more selected from the group consisting of citric acid, acetic acid, formic acid, fumaric acid, lactic acid, and propionic acid. Specifically, the organic acid may be citric acid.

The chelating agent and the organic acid may each independently be included in an amount of 0.1 part by weight to 2 parts by weight with respect to 100 parts by weight of the base resin, specifically, in an amount of 0.1 part by weight or more, 0.3 parts by weight or more, 0.5 parts by weight or more, 0.7 parts by weight or more, or 1 part by weight or more, and 2 parts by weight or less, 1.7 parts by weight or less, 1.5 parts by weight or less, or 1.3 parts by weight or less with respect to 100 parts by weight of the base resin. When used in the above range of content, the deodorizing ability of the superabsorbent polymer may be further improved without deteriorating the absorption properties.

The chelating agent may be used after being mixed in the form of a salt with an aqueous solution, and thus the above range of content is based on the solid content.

According to still another aspect of the present invention, the superabsorbent polymer includes a base resin complex including a base resin, in which an acrylic acid-based monomer having acidic groups of which at least part is neutralized, and an internal crosslinking agent are polymerized, and at least part of a deodorant which is supported on the polymer chains of the base resin; and

a surface crosslinked layer which is formed on the surface of the base resin,
wherein the deodorant includes a metal organic framework (MOF) or an ion exchange resin.

The base resin complex is in a state where a part of the deodorant particles are stuck to be supported in the base resin chains through surface crosslinking while the base resin and the deodorant are physically mixed, resulting from the surface crosslinking reaction after mixing the base resin with the deodorant, metal organic framework (MOF) or ion exchange resin. Therefore, the bonded state is maintained without being easily detached until subsequent processes or the completion of products such as diapers, etc., and when wet by urine which causes bad odor, the superabsorbent polymer expands to desorb the deodorant which is then widely distributed, thereby exerting deodorizing ability.

With regard to the base resin, the raw materials used in the base resin, including the acrylic acid-based monomer and the internal crosslinking agent, and the preparation method are as described in the method of preparing the superabsorbent polymer.

Further, the type, content, and characteristics of the deodorant are as described in the method of preparing the superabsorbent polymer.

Further, the surface crosslinking agent that forms the surface crosslinked layer, and the preparation method are as described in the method of preparing the superabsorbent polymer.

For example, the MOF includes one or more metal ions selected from the group consisting of Zn, Ti, Co, Al, and Zr, and one or more organic ligands selected from the group consisting of imidazole, alkylimidazole, alkoxyimidazole, terephthalic acid, and aminoterephthalic acid.

Further, the ion exchange resin includes a polystyrene-divinylbenzene-based, polymethacrylate-divinylbenzene-based, or polyacrylate-divinylbenzene-based resin.

Further, the deodorant may be mixed in an amount of 1 part by weight to 10 parts by weight with respect to 100 parts by weight of the base resin.

The superabsorbent polymer according to one embodiment of the present invention may further include a chelating agent or an organic acid in order to improve the deodorizing ability.

The chelating agent may include one or more selected from the group consisting of ethylenediaminetetraacetic acid (EDTA), L-glutamic acid diacetic acid (GLDA), methyl glycine diacetic acid (MGDA), hydroxyethyl ethylenediaminetriacetic acid (HEDTA), ethanol diglycinic acid (EDG), diethylenetriaminepentaacetic acid (DTPA), and salts thereof. More specifically, the chelating agent may be ethylenediaminetetraacetic acid (EDTA), or L-glutamic acid diacetic acid (GLDA).

The organic acid may be one or more selected from the group consisting of citric acid, acetic acid, formic acid, fumaric acid, lactic acid, and propionic acid. Specifically, the organic acid may be citric acid.

The chelating agent and the organic acid may each independently be included in an amount of 0.1 part by weight to 2 parts by weight with respect to 100 parts by weight of the base resin, specifically, in an amount of 0.1 part by weight or more, 0.3 parts by weight or more, 0.5 parts by weight or more, 0.7 parts by weight or more, or 1 part by weight or more, and 2 parts by weight or less, 1.7 parts by weight or less, 1.5 parts by weight or less, or 1.3 parts by weight or less with respect to 100 parts by weight of the base resin. When used in the above range of content, the deodorizing ability of the superabsorbent polymer may be further improved without deteriorating the absorption properties.

The chelating agent may be used after being mixed in the form of a salt with an aqueous solution, and thus the above range of content is based on the solid content.

Hereinafter, preferred exemplary embodiments will be provided for better understanding of the present invention. However, the following exemplary embodiments are provided only for illustrating the present invention, but the present invention is not limited thereto.

**<Example>**

**Example 1**

100 g of acrylic acid, 0.37 g of N,N'-methylenebisacrylamide as a crosslinking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer solution composition having a monomer concentration of 45.8% by weight. Then, the aqueous monomer solution composition was fed into a feed zone of a polymerization reactor which was equipped with a continuously moving conveyor belt, and then UV was irradiated (irradiation amount: 10 mW/cm$^2$) with a UV irradiation device while maintaining the polymerization atmosphere temperature at 80°C, and UV polymerization was performed for 2 minutes to prepare a hydrogel polymer.

The hydrogel polymer was transferred to a meat chopper and chopped to 2 mm to 10 mm. At this time, the chopped hydrogel polymer had a water content of 47% by weight. Subsequently, the hydrogel polymer was dried in a hot air dryer of 170°C for 30 minutes, and the dried hydrogel polymer was pulverized with a pin mill, and then, classified using a sieve to obtain a polymer having a particles size of 150 μm to 850 μm, and thus a base resin having a water content of 1% by weight or less was prepared.

10 parts by weight of Zn-MOF (ZIF-8, Zn$^{2+}$+2-methylimidazole) as a deodorant was mixed with respect to 100 parts by weight of the base resin prepared above while stirring in a dry state.

Thereafter, the mixture in which the deodorant was mixed, was evenly mixed with a surface crosslinking solution (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) with respect to 100 parts by weight of the base resin.

Next, a surface crosslinking reaction was performed on the mixture at 140°C for 30 minutes. After completing the surface crosslinking reaction, a superabsorbent polymer having a particle size of 150 μm to 850 μm was obtained by classification using a sieve.

**Example 2**

A superabsorbent polymer was prepared in the same manner as in Example 1, except that 5 parts by weight of Zn-MOF (ZIF-8, Zn$^{2+}$+2-methylimidazole) as a deodorant was mixed with respect to 100 parts by weight of the base resin prepared above while stirring in a dry state in Example 1.

**Example 3**

A superabsorbent polymer was prepared in the same manner as in Example 1, except that 2 parts by weight of Zn-MOF (ZIF-8, Zn$^{2+}$+2-methylimidazole) as a deodorant was mixed with respect to 100 parts by weight of the base resin prepared above while stirring in a dry state in Example 1.

## Example 4

A superabsorbent polymer was prepared in the same manner as in Example 1, except that 10 parts by weight of Ti-MOF ($Tn^{2+}$+2-methylimidazole) as a deodorant was mixed with respect to 100 parts by weight of the base resin prepared above while stirring in a dry state in Example 1.

## Example 5

A superabsorbent polymer was prepared in the same manner as in Example 1, except that 10 parts by weight of an ion exchange resin Amberlite XAD4 (available from AIFA) was mixed with respect to 100 parts by weight of the base resin prepared above while stirring in a dry state in Example 1.

## Example 6

The preparation of a base resin was performed in the same manner as in Example 1.

5 parts by weight of Zn-MOF (ZIF-8, $Zn^{2+}$+2-methylimidazole) as a deodorant was mixed with respect to 100 parts by weight of the base resin prepared above while stirring in a dry state.

Thereafter, the mixture in which the deodorant was mixed, was evenly mixed with a surface crosslinking solution (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) with respect to 100 parts by weight of the base resin.

Next, a surface crosslinking reaction was performed on the mixture at 140°C for 30 minutes. After the surface treatment, an aqueous citric acid solution was prepared, in which citric acid was included in an amount of 5 parts by weight with respect to 100 parts by weight of the base resin, and mixed with the superabsorbent polymer by spraying thereto, followed by drying at 80°C for 25 minutes. Then, a superabsorbent polymer having a particle size of 150 $\mu$m to 850 $\mu$m was obtained by classification using a sieve.

## Example 7

The preparation of a base resin was performed in the same manner as in Example 1.

5 parts by weight of Zn-MOF (ZIF-8, $Zn^{2+}$+2-methylimidazole) as a deodorant was mixed with respect to 100 parts by weight of the base resin prepared above while stirring in a dry state.

Thereafter, the mixture in which the deodorant was mixed, was evenly mixed with a surface crosslinking solution (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) with respect to 100 parts by weight of the base resin.

Next, a surface crosslinking reaction was performed on the mixture at 140°C for 30 minutes. After completing the surface crosslinking reaction, an aqueous EDTA solution was prepared, in which EDTA was included in an amount of 5 parts by weight with respect to 100 parts by weight of the base resin, and mixed with the superabsorbent polymer by spraying thereto, followed by drying at 80°C for 25 minutes. Then, a superabsorbent polymer having a particle size of 150 $\mu$m to 850 $\mu$m was obtained by classification using a sieve.

## Comparative Example 1

100 g of acrylic acid, 0.37 g of N,N'-methylenebisacrylamide as a crosslinking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer solution composition having a monomer concentration of 45.8% by weight. Then, the aqueous monomer solution composition was fed into a feed zone of a polymerization reactor which was equipped with a continuously moving conveyor belt, and then UV was irradiated (irradiation amount: 10 mW/cm$^2$) with a UV irradiation device while maintaining the polymerization atmosphere temperature at 80°C, and UV polymerization was performed for 2 minutes to prepare a hydrogel polymer.

The hydrogel polymer was transferred to a meat chopper and chopped to 2 mm to 10 mm. At this time, the chopped hydrogel polymer had a water content of 47% by weight. Subsequently, the hydrogel polymer was dried in a hot air dryer of 170°C for 30 minutes, and the dried hydrogel polymer was pulverized with a pin mill, and then, classified using a sieve to obtain a polymer having a particles size of 150 $\mu$m to 850 $\mu$m, and thus a base resin having a water content of 1% by weight or less was prepared.

Thereafter, a surface crosslinking solution (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) was evenly mixed with respect to 100 parts by weight of the base resin.

Next, a surface crosslinking reaction was performed on the mixture at 140°C for 30 minutes. After completing the surface crosslinking reaction, a superabsorbent polymer having a particle size of 150 $\mu$m to 850 $\mu$m was obtained by classification using a sieve.

## Comparative Example 2

In Comparative Example 1, after completing the surface crosslinking reaction, an aqueous EDTA solution was prepared, in which EDTA was included in an amount of 1 part by weight with respect to 100 parts by weight of the base resin, and mixed with the superabsorbent polymer by spraying thereto, followed by drying at 80°C for 25 minutes. Then, a superabsorbent polymer having a particle size of 150 $\mu$m to 850 $\mu$m was obtained by classification using a sieve.

## Comparative Example 3

In Comparative Example 1, after completing the surface crosslinking reaction, an aqueous citric acid solution was prepared, in which citric acid was included in an amount of 5 part by weight with respect to 100 parts by weight of the base resin, and mixed with the superabsorbent polymer by spraying thereto, followed by drying at 80°C for 25 minutes. Then, a superabsorbent polymer having a particle size of 150 $\mu$m to 850 $\mu$m was obtained by classification using a sieve.

## Comparative Example 4

A superabsorbent polymer was prepared in the same manner as in Example 1, except that 5 parts by weight of volcanic ash as a deodorant was mixed with respect to 100 parts by weight of the base resin prepared above while stirring in a dry state in Example 1.

## Comparative Example 5

A superabsorbent polymer was prepared in the same manner as in Example 1, except that 10 parts by weight of laponite (XLS) as a deodorant was mixed with respect to 100 parts by weight of the base resin prepared above while stirring in a dry state in Example 1.

The main features of Examples and Comparative Examples are summarized in Table 1.

[Table 1]

| | | Deodorant | | | Additive | | |
|---|---|---|---|---|---|---|---|
| | | Type | Content* | Time point of feeding | Type | Content* | Time point of feeding |
| | Example 1 | Zn-MOF | 10 | Mixed with base resin before surface crosslinking | - | | |
| | Example 2 | Zn-MOF | 5 | Mixed with base resin before surface crosslinking | - | | |
| | Example 3 | Zn-MOF | 2 | Mixed with base resin before surface crosslinking | - | | |
| | Example 4 | Ti-MOF | 10 | Mixed with base resin before surface crosslinking | - | | |
| | Example 5 | Ion exchange resin | 10 | Mixed with base resin before surface crosslinking | - | | |
| | Example 6 | Zn-MOF | 5 | Mixed with base resin before surface crosslinking | Citric acid | 5 | After surface crosslinking |
| | Example 7 | Zn-MOF | 5 | Mixed with base resin before surface crosslinking | EDTA | 1 | After surface crosslinking |

(continued)

| | Deodorant | | | Additive | | |
|---|---|---|---|---|---|---|
| | Type | Content* | Time point of feeding | Type | Content* | Time point of feeding |
| Comparative Example 1 | - | | | - | | |
| Comparative Example 2 | - | | | EDTA | 1 | After surface crosslinking |
| Comparative Example 3 | - | | | Citric acid | 5 | After surface crosslinking |
| Comparative Example 4 | Volcanic ash | 5 | Mixed with base resin before surface crosslinking | | | |
| Comparative Example 5 | Laponite | 10 | Mixed with base resin before surface crosslinking | | | |
| (In Table 1, the contents of each deodorant and additive are expressed as parts by weight with respect to 100 parts by weight of the base resin.) | | | | | | |

**Experimental Example**

Each physical property was measured for the superabsorbent polymers prepared in Examples and Comparative Examples by the following methods.

Unless otherwise indicated, all evaluations of the following physical properties were conducted at constant temperature and humidity ($23 \pm 1$°C, relative humidity of $50 \pm 10$%), and a physiological saline or saline solution refers to 0.9 wt% aqueous sodium chloride (NaCl) solution.

Further, unless otherwise indicated, the evaluation of the physical properties of the superabsorbent polymers was performed on resins having a particle size of 150 $\mu$m to 850 $\mu$m that were classified through an ASTM standard sieve.

**(1) Centrifuge Retention Capacity (CRC)**

The water retention capacity by absorbency under no load was measured for the superabsorbent polymers of Examples and Comparative Examples in accordance with European Disposables and Nonwovens Association (EDANA) standard EDANA WSP 241.3.

In detail, the superabsorbent polymer $W_0(g)$ (about 0.2 g) obtained through Examples and Comparative Examples was evenly put in a nonwoven fabric-made bag, followed by sealing. Then, the bag was immersed in a physiological saline solution (0.9 wt%) at room temperature. After 30 minutes, water was removed from the bag using a centrifuge at 250 G for 3 minutes, and the weight $W_2(g)$ of the bag was then measured. Further, the same procedure was carried out without using the resin, and then the resultant weight $W_1(g)$ was measured.

By using the respective weights thus obtained, CRC (g/g) was calculated according to the following Equation 1.

[Equation 1]

$$CRC \ (g/g) = \{[W_2(g) - W_1(g)]/W_0(g)\} - 1$$

**(2) Absorbency under Pressure (AUP)**

Absorbency under pressure of 0.7 psi of the superabsorbent polymers of Examples and Comparative Examples was measured in accordance with the EDANA method WSP 242.3.

First, when measuring absorbency under pressure, the classified polymer used at the time of CRC measurement was used.

In detail, a 400 mesh stainless steel net was installed in the bottom of a plastic cylinder having an internal diameter of 25 mm. The superabsorbent polymer $W_0(g)$ was uniformly scattered on the stainless steel net under conditions of room temperature and humidity of 50%. A piston capable of uniformly providing a load of 0.7 psi was placed thereon, in which an external diameter of the piston was slightly smaller than 25 mm, there was no gab between the internal wall of the cylinder and the piston, and the jig-jog of the cylinder was not interrupted. At this time, the weight $W_3(g)$ of the apparatus was measured.

After placing a glass filter having a diameter of 90 mm and a thickness of 5 mm in a petri dish having a diameter of 150 mm, a physiological saline solution consisting of 0.9% by weight of sodium chloride was poured until the surface level of the physiological saline solution became equal to the upper surface of the glass filter. A sheet of filter paper having a diameter of 90 mm was placed on the glass filter. The measurement apparatus was mounted on the filter paper, thereby getting the liquid absorbed under the load for 1 hour. 1 hour later, after lifting the measurement apparatus up, the weight $W_4$ (g) was measured. Absorbency under pressure (g/g) was calculated using the obtained weights according to the following Equation 2.

[Equation 2]

$$AUP(g/g) = [W_4(g) - W_3(g)]/W_0(g)$$

### (3) Deodorizing Ability (sorbent tube test)

Guaiacol as a phenolic compound, dimethyl trisulfide as a sulfur compound, and 3-methyl butanal as an aldehyde compound were selected as odorous substances, and the deodorizing ability was tested using a sorbent tube test.

- Sorbent tube test method: 1 g of the superabsorbent polymer was put in a 500 mL glass bottle, and then 25 mL of the odorous substance was injected. Thereafter, aging was performed for 3 hours in a constant temperature chamber and collection was performed for 20 minutes. At this time, the temperature of the constant temperature chamber was 35°C and the $N_2$ flow rate was 250 mL/min. The bad odor pushed out was then adsorbed to the connected sorbent tube (TENAX/GR), which was collected twice for the same sample. The collection results were analyzed by GC to confirm the results.

-

   Deodorizing ability (%) = (Amount of odor of reference sample (sample of Comparative Example 1) measured by GC - Amount of odor of sample measured by GC) / Amount of odor of reference sample (sample of Comparative Example 1) measured by GC X 100(%)

### (4) Color Test

A color test was performed by weighing 5 g of the superabsorbent polymer on an aluminum dish, spreading the same thinly, and measuring using a color difference meter Labscan XE (HunterLab's product). The measured values were output as three parameters: L, a, and b. These values were substituted into the following equation to calculate the color evaluation value (WI; Whiteness Index). At this time, as the WI value is higher, the color is closer to white.

$$WI(\text{Whiteness Index}) = 100 - \sqrt{(100 - L)^2 + x^2 + y^2}$$

The results of the experiments are shown in Table 2. The values of the deodorizing ability in Table 2 show the deodorizing efficiency, relative to that of Comparative Example 1, and the higher deodorizing efficiency has the higher value.

[Table 2]

| | CRC (unit: g/g) | 0.7 psi AUP (unit: g/g) | Deodorizing ability (sorbent tube test) (unit: %) | | | | Color test (WI) |
|---|---|---|---|---|---|---|---|
| | | | Aldehyde | Phenol | Sulfur | Mean | |
| Example 1 | 36.2 | 16.2 | 45 | 41 | 81 | 55.7 | 75.0 |
| Example 2 | 36.3 | 16.5 | 21 | 49 | 65 | 45 | 75.5 |
| Example 3 | 36.8 | 16.8 | 10 | 29 | 31 | 23.3 | 75.6 |
| Example 4 | 35.9 | 16.1 | 19 | 32 | 66 | 39 | 74.9 |
| Example 5 | 36.0 | 16.3 | 51 | 71 | 95 | 72.3 | 75.1 |

(continued)

| | CRC (unit: g/g) | 0.7 psi AUP (unit: g/g) | Deodorizing ability (sorbent tube test) (unit: %) | | | | Color test (WI) |
|---|---|---|---|---|---|---|---|
| | | | Aldehyde | Phenol | Sulfur | Mean | |
| Example 6 | 36.2 | 15.9 | 32 | 51 | 73 | 52 | 73.1 |
| Example 7 | 36.1 | 15.8 | 31 | 50 | 72 | 51 | 76.0 |
| Comparative Example 1 | 37.3 | 18.0 | 0 | 0 | 0 | 0 | 76.1 |
| Comparative Example 2 | 37.0 | 17.0 | 0 | 0 | 20 | 6.7 | 76.1 |
| Comparative Example 3 | 37.3 | 16.8 | 15 | 0 | 21 | 12 | 72.2 |
| Comparative Example 4 | 35.9 | 15.1 | 33 | 54 | 45 | 44 | 43.4 |
| Comparative Example 5 | 36.2 | 16.0 | 8 | 16 | 18 | 14 | 76.0 |

Referring to Table 2, the superabsorbent polymers prepared by mixing with the MOF or ion exchange resin as a deodorant before surface crosslinking according to the preparation method of the present invention showed the excellent deodorizing ability evenly against phenolic, sulfur, and aldehyde compounds, which are the main causes of bad odor, and also showed the excellent color characteristics.

It was confirmed that Comparative Examples 2, 3, and 5, in which only the commonly used deodorant, EDTA, organic acids, or laponite was used, showed the poor deodorizing ability, and Comparative Example 4, in which volcanic ash was used as a deodorant adsorbent, had inferior color characteristics and was not suitable for application to diapers.

**Claims**

1.  A method of preparing a superabsorbent polymer, the method comprising the steps of:

    forming a hydrogel polymer by performing a polymerization on a monomer composition including an acrylic acid-based monomer having acidic groups of which at least part is neutralized, an internal crosslinking agent, and a polymerization initiator (step 1);
    forming a base resin by drying, pulverizing, and classifying the hydrogel polymer (step 2);
    mixing the base resin and a deodorant (step 3); and
    performing a surface crosslinking reaction on the mixture of the base resin and the deodorant in the presence of a surface crosslinking solution including a surface crosslinking agent (step 4),
    wherein the deodorant includes a metal organic framework (MOF) or an ion exchange resin.

2.  The method of claim 1, wherein the MOF includes one or more metal ions selected from the group consisting of Zn, Ti, Co, Al, and Zr, and one or more organic ligands selected from the group consisting of imidazole, alkylimidazole, alkoxyimidazole, terephthalic acid, and aminoterephthalic acid.

3.  The method of claim 1, wherein the ion exchange resin includes a polystyrene-divinylbenzene-based, polymethacrylate-divinylbenzene-based, or polyacrylate-divinylbenzene-based resin.

4.  The method of claim 1, wherein the deodorant is mixed in an amount of 1 part by weight to 10 parts by weight with respect to 100 parts by weight of the base resin.

5.  The method of claim 1, wherein a chelating agent or an organic acid is further mixed at or after the step 4.

6.  The method of claim 5, wherein the chelating agent includes one or more selected from the group consisting of ethylenediaminetetraacetic acid (EDTA), L-glutamic acid diacetic acid (GLDA), methyl glycine diacetic acid (MGDA), hydroxyethyl ethylenediaminetriacetic acid (HEDTA), ethanol diglycinic acid (EDG), diethylenetriaminepentaacetic acid (DTPA), and salts thereof.

7.  The method of claim 5, wherein the organic acid includes one or more selected from the group consisting of citric acid, acetic acid, formic acid, fumaric acid, lactic acid, and propionic acid.

8. The method of claim 5, wherein the chelating agent or the organic acid is each independently mixed in an amount of 0.1 part by weight to 5 parts by weight with respect to 100 parts by weight of the base resin.

9. A superabsorbent polymer comprising:

a base resin complex including a base resin, in which an acrylic acid-based monomer having acidic groups of which at least part is neutralized, and an internal crosslinking agent are polymerized, and at least part of a deodorant which is supported on the polymer chains of the base resin; and
a surface crosslinked layer which is formed on the surface of the base resin,
wherein the deodorant includes a metal organic framework (MOF) or an ion exchange resin.

10. The superabsorbent polymer of claim 9, wherein the MOF includes one or more metal ions selected from the group consisting of Zn, Ti, Co, Al, and Zr, and one or more organic ligands selected from the group consisting of imidazole, alkylimidazole, alkoxyimidazole, terephthalic acid, and aminoterephthalic acid.

11. The superabsorbent polymer of claim 9, wherein the ion exchange resin includes a polystyrene-divinylbenzene-based, polymethacrylate-divinylbenzene-based, or polyacrylate-divinylbenzene-based resin.

12. The superabsorbent polymer of claim 9, wherein the deodorant is mixed in an amount of 1 part by weight to 10 parts by weight with respect to 100 parts by weight of the base resin.

13. The superabsorbent polymer of claim 9, further comprising a chelating agent or an organic acid.

14. The superabsorbent polymer of claim 13, wherein the chelating agent includes one or more selected from the group consisting of ethylenediaminetetraacetic acid (EDTA), L-glutamic acid diacetic acid (GLDA), methyl glycine diacetic acid (MGDA), hydroxyethyl ethylenediaminetriacetic acid (HEDTA), ethanol diglycinic acid (EDG), diethylenetriaminepentaacetic acid (DTPA), and salts thereof.

15. The superabsorbent polymer of claim 13, wherein the organic acid includes one or more selected from the group consisting of citric acid, acetic acid, formic acid, fumaric acid, lactic acid, and propionic acid.

## INTERNATIONAL SEARCH REPORT

| International application No. |
| --- |
| **PCT/KR2024/095142** |

### A. CLASSIFICATION OF SUBJECT MATTER

**C08J 3/24**(2006.01)i; **C08J 3/12**(2006.01)i; **C08F 20/10**(2006.01)i; **C08L 33/04**(2006.01)i; **C08L 25/08**(2006.01)i; **C08L 25/14**(2006.01)i; **C08K 5/56**(2006.01)i; **C08K 5/00**(2006.01)i; **C08K 5/09**(2006.01)i; **C08K 5/17**(2006.01)i

According to International Patent Classification (IPC) or to both national classification and IPC

### B. FIELDS SEARCHED

Minimum documentation searched (classification system followed by classification symbols)

C08J 3/24(2006.01); A01K 1/015(2006.01); A61L 9/01(2006.01); A61L 9/012(2006.01); A61L 9/014(2006.01); A61L 9/16(2006.01); B01J 20/20(2006.01); C08K 3/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorbent polymer), 소취(deodor), 킬레이트제(chelator), 유기산(organic acid), 금속 유기 골격체(metal organic framework, MOF), 이온교환수지(ion exchange resin), 아크릴산계 단량체(acrylic acid monomer), 표면 가교제(surface crosslinker), 내부가교제(internal crosslinker), 분쇄(grinding), 함수 겔 중합체(hydrogel polymer)

### C. DOCUMENTS CONSIDERED TO BE RELEVANT

| Category* | Citation of document, with indication, where appropriate, of the relevant passages | Relevant to claim No. |
| --- | --- | --- |
| Y | KR 10-2019-0035313 A (LG CHEM, LTD.) 03 April 2019 (2019-04-03)<br>See claims 1, 3-6 and 8; and paragraphs [0040] and [0044]. | 1-15 |
| Y | JP 2019-088499 A (OHARA PARAGIUM CHEMICAL CO., LTD.) 13 June 2019 (2019-06-13)<br>See claims 1 and 6; and paragraphs [0007], [0018]-[0021], [0051] and [0062]. | 1,2,4-10,12-15 |
| Y | JP 2017-205225 A (ORGANO KK) 24 November 2017 (2017-11-24)<br>See claims 1 and 5; and paragraphs [0006] and [0057]. | 1,3-9,11-15 |
| A | KR 10-2005-0081606 A (CHOI, Yong Suk et al.) 19 August 2005 (2005-08-19)<br>See entire document. | 1-15 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 May 2024** | **24 May 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/095142** |

| C. | DOCUMENTS CONSIDERED TO BE RELEVANT | |
| --- | --- | --- |
| Category* | Citation of document, with indication, where appropriate, of the relevant passages | Relevant to claim No. |
| A | KR 10-2021-0001839 A (KOREA INSTITUTE OF INDUSTRIAL TECHNOLOGY) 06 January 2021 (2021-01-06)<br>See entire document. | 1-15 |

Form PCT/ISA/210 (second sheet) (July 2022)

## INTERNATIONAL SEARCH REPORT
### Information on patent family members

International application No.

**PCT/KR2024/095142**

| Patent document cited in search report | | | Publication date (day/month/year) | Patent family member(s) | | | Publication date (day/month/year) |
|---|---|---|---|---|---|---|---|
| KR | 10-2019-0035313 | A | 03 April 2019 | KR | 10-2487978 | B1 | 11 January 2023 |
| JP | 2019-088499 | A | 13 June 2019 | | None | | |
| JP | 2017-205225 | A | 24 November 2017 | JP | 6690990 | B2 | 28 April 2020 |
| KR | 10-2005-0081606 | A | 19 August 2005 | KR | 10-0572277 | B1 | 19 April 2006 |
| KR | 10-2021-0001839 | A | 06 January 2021 | KR | 10-2289660 | B1 | 17 August 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230019560

- KR 1020240020382

### Non-patent literature cited in the description

- **REINHOLD SCHWALM**. UV Coatings: Basics, Recent Developments and New Application. Elsevier, 2007, 115

- **ODIAN**. Principle of Polymerization. Wiley, 1981, 203